(19) Europäisches Patentamt
European Patent Office
Office européen des brevets

(11) EP 3 662 812 A1

(12) EUROPÄISCHE PATENTANMELDUNG

(43) Veröffentlichungstag:
10.06.2020 Patentblatt 2020/24

(51) Int Cl.:
**A61B 3/10** (2006.01)

(21) Anmeldenummer: 18210058.6

(22) Anmeldetag: 04.12.2018

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **HAAG-STREIT AG**
**CH-3098 Köniz (CH)**

(72) Erfinder:
• **Wagner, Jörg**
  **3013 Bern (CH)**
• **Cattin, Philippe**
  **5210 Windisch (CH)**
• **Robledo, Lucio**
  **3013 Bern (CH)**

(74) Vertreter: **Glawe, Delfs, Moll**
**Partnerschaft mbB von**
**Patent- und Rechtsanwälten**
**Postfach 13 03 91**
**20103 Hamburg (DE)**

(54) **VERFAHREN ZUM DURCHFÜHREN EINER BEWEGUNGSKORREKTUR BEI AUGENMESSUNGEN UND MESSSYSTEM**

(57) Verfahren zum Durchführen einer Bewegungskorrektur bei Messungen an einem menschlichen Auge (14), bei dem das Auge (14) mit einem Messstrahl (15) gescannt wird, um einen Satz von Positionswerte (39) einer Augenstruktur(40) zu gewinnen. Das Scannen erstreckt sich über einen Messzeitraum, wobei die Positionswerte (39) jeweils mit einem Zeitstempel innerhalb des Messzeitraums versehen sind. Es werden eine Flächenform und eine Verschiebungsfunktion (38) ermittelt, wobei die Verschiebungsfunktion (38) einen Bewegungsablauf während des Messzeitraums darstellt, und wobei die Flächenform und die Verschiebungsfunktion (38) derart ermittelt werden, dass die Positionswerte (39) durch die Flächenform angenähert werden, wenn die Flächenform gemäß der Verschiebungsfunktion (38) bewegt wird. Die Erfindung betrifft außerdem ein zugehöriges Messsystem.

Fig. 1

EP 3 662 812 A1

**Beschreibung**

Die Erfindung betrifft ein Verfahren zum Durchführen einer Bewegungskorrektur bei Augenmessungen. Die Erfindung betrifft außerdem ein zugehöriges Messsystem.

Bei Messungen am Auge kann das Messergebnis verfälscht werden, wenn sich das Auge während der Aufnahme der Messwerte bewegt. Je mehr Zeit die Messung in Anspruch nimmt, desto stärker wirken die Bewegungen des Auges sich aus. Eine längere Messdauer ergibt sich insbesondere beim Abtasten des Auges mit einem Messstrahl, wie es beispielsweise bei OCT-Messungen üblicherweise der Fall ist.

Eine mögliche Vorgehensweise ist es, zunächst eine schnelle Referenzmessung über den Messbereich aufzunehmen, bei der die Messdauer so kurz ist, dass die Messung nicht durch Bewegungen des Auges verfälscht wird. Die nachfolgenden längeren Messungen mit einer größeren Anzahl von Stützpunkten, können anhand der ersten Messung ausgerichtet werden, um die Bewegung aus den Daten zu eliminieren, US 7,365,856 B2, EP 2 797 493 A1. Nachteil bei dieser Vorgehensweise ist, dass durch eine Ungenauigkeit bei der Referenzmessung das gesamte Messergebnis verfälscht werden kann. Außerdem ist ein schnelles Scansystem vonnöten um die Referenzmessung genügend schnell ausführen zu können.

Alternativ können auch mehrere schnelle Messungen aufgenommen werden welche danach aufeinander ausgerichtet werden. Voraussetzung dafür ist, dass innerhalb einer Messung eine sinnvolle Geometrie der Auges (z.B. ein Meridian) genügend schnell aufgenommen wird so dass die Augenbewegung darin vernachlässigt werden kann. Auch dafür ist ein schnelles System mit schneller Datenerfassung und schnellem Scanner erforderlich.

Der Erfindung liegt die Aufgabe zugrunde, ein Verfahren und ein Messsystem zum Durchführen einer Bewegungskorrektur bei Augenmessungen vorzustellen, die eine verminderte Fehleranfälligkeit haben. Ausgehend vom genannten Stand der Technik wird die Aufgabe gelöst mit den Merkmalen der unabhängigen Ansprüche. Vorteilhafte Ausführungsformen sind in den Unteransprüchen angegeben.

Bei dem erfindungsgemäßen Verfahren wird das Auge mit einem Messstrahl gescannt, um einen Satz von Positionswerten einer Augenstruktur zu gewinnen. Das Scannen erstreckt sich über einen Messzeitraum, wobei die Positionswerte jeweils mit einem Zeitstempel innerhalb des Messzeitraums versehen sind. Es werden eine Flächenform und eine Verschiebungsfunktion ermittelt, wobei die Verschiebungsfunktion einen Bewegungsablauf während des Messzeitraums darstellt. Die Flächenform und die Verschiebungsfunktion werden derart ermittelt, dass die Positionswerte durch die Flächenform angenähert werden, wenn die Flächenform gemäß der Verschiebungsfunktion bewegt wird.

Die Erfindung geht von der Annahme aus, dass die Bewegung, die das Auge innerhalb des Messzeitraums durchgeführt hat, durch eine Verschiebungsfunktion beschrieben werden kann. In einem Idealfall ist die Messung frei von Messfehlern, es wird eine Flächenform gefunden, die der tatsächlichen Form der Augenstruktur entspricht, und es wird eine Verschiebungsfunktion gefunden, die dem tatsächlichen Bewegungsablauf des Auges entspricht. Verschiebt man diese Flächenform entsprechend dieser Verschiebungsfunktion, so liegen (bei geeigneter gegenseitiger Anpassung des räumlichen und zeitlichen Koordinatensystems) alle von der Augenstruktur aufgenommenen Positionswerte auf der Flächenform. Im Unterschied zum Stand der Technik ist eine Referenzmessung als Ausgangspunkt für die Bewegungskorrektur nicht erforderlich. Dies ist von besonderem Vorteil, wenn das Scansystem nicht über die nötige Geschwindigkeit verfügt um eine bewegungsarme Referenzmessung durchzuführen.

In der Praxis treten Messfehler auf, und es kommt zu Abweichungen sowohl zwischen der Flächenform und der tatsächlichen Form der Augenstruktur als auch zwischen der Verschiebungsfunktion und dem tatsächlichen Bewegungsablauf. Mit der Formulierung, dass die Positionswerte durch die Flächenform "angenähert" werden, wird diese Abweichung zwischen idealem und praktisch durchgeführtem Verfahren berücksichtigt. Bei Durchführung des Verfahrens in idealer Form würden die Positionswerte und die Flächenform identisch übereinstimmen. Annähern im Sinne der Erfindung bedeutet, die Flächenform und die Verschiebungsfunktion so zu ermitteln, dass die Positionswerte im Rahmen der Randbedingungen der praktisch durchgeführten Messung angenähert werden. Beispielsweise kann ein Gütekriterium für die Annäherung vorgegeben werden, und es kann die Flächenform und die Verschiebungsfunktion so ermittelt werden, dass die anhand des Gütekriteriums festgestellte Abweichung kleiner ist als ein vorgegebener Schwellwert. Wenn in der nachfolgenden Beschreibung von Positionswerten gesprochen wird, die auf der Flächenform liegen, so ist dies gleichbedeutend mit einer Annäherung in diesem Sinne.

Betrachtet man einen ersten Zeitpunkt und einen zweiten Zeitpunkt innerhalb des Messzeitraums, so ergibt sich aus der Verschiebungsfunktion ein Bewegungsablauf, der die Bewegung des Auges zwischen den beiden Zeitpunkten beschreibt. Eine Flächenform, die die tatsächliche Form der Augenstruktur richtig wiedergibt, und die gemäß der Verschiebungsfunktion verschoben wird, kann gemeinsam mit den bei der Messung gewonnenen Positionswerten so in einem Koordinatensystem dargestellt werden, dass für beide Zeitpunkte die Flächenform auf dem mit der Messung gewonnenen Positionswert liegt. Gemeinsame Darstellung der Flächenform und der Positionswerte in einem Koordinatensystem bedeutet, dass die räumlichen Koordinaten der Flächenform und der Positionswerte richtig zueinander ausgerichtet ist und dass der zeitliche Ablauf der Verschiebungsfunktion mit den Zeitstempeln der Positionswerte abgeglichen ist. Die Formulierung, dass die Flächenform und die Positionswerte entsprechend der Verschiebungsfunktion relativ zueinander verschoben werden, beinhaltet keine Einschränkung auf eine bestimmte Darstellungsform der räumlichen und zeitlichen Koordinaten. Möglich sind verschiedene Formen von Bewegungsdarstellungen, Differenzangaben über der Zeit und Ähnliches. Es kann ein Zeitpunkt innerhalb des Messzeitraums als Referenzzeitpunkt ausgewählt werden, ausgehend von dem die relativen Koordinaten definiert sind.

Das Verfahren kann so durchgeführt werden, dass alle Positionswerte, die einen Zeitstempel zwischen dem ersten Zeitpunkt und dem zweiten Zeitpunkt haben, auf der gemäß der Verschiebungsfunktion verschobenen Flächenform liegen. Die Anzahl der Positionswerte zwischen dem ersten Zeitpunkt und dem zweiten Zeitpunkt kann größer sein als 1000, vorzugsweise größer sein als 5000, weiter vorzugsweise größer sein als 20.000. Der erste Zeitpunkt kann mit dem Beginn des Messzeitraums zusammenfallen. Der zweite Zeitpunkt kann mit dem Ende des Messzeitraums zusammenfallen. Möglich ist auch, dass der Zeitraum zwischen dem ersten Zeitpunkt und im zweiten Zeitpunkt einem Abschnitt des Messzeitraums entspricht.

Das Ermitteln der Flächenform und/oder das Ermitteln der Verschiebungsfunktion kann darin bestehen, dass verschiedene Flächenformen bzw. Verschiebungsfunktionen ausprobiert werden und anhand des Gütekriteriums festgestellt wird, durch welche Kombination aus Flächenform und Verschiebungsfunktion die Positionswerte am besten angenähert werden. Dabei können alle in dem Messzeitraum aufgenommenen Positionswerte angenähert werden oder eine Teilmenge aus diesen Positionswerten. Die Flächenform und Verschiebungsfunktion, bei denen die Abweichung zu den Positionswerten am kleinsten wird, können als bestmögliche Annäherung an die Positionswerte angenommen werden. Die betreffenden Flächenformen und/oder Verschiebungsfunktionen können im Rahmen eines Optimierungsverfahrens ausgewählt werden. Die Flächenformen und/oder Verschiebungsfunktion können beispielsweise zufällig ausgewählt werden, und es kann nach der Methode der kleinsten Quadrate festgestellt werden, welche Kombination aus Verschiebungsfunktion und Flächenform die Positionswerte am besten annähert.

Die so ermittelte Flächenform kann als bewegungskorrigierte Repräsentation der Form der abgetasteten Fläche angesehen werden und als Ergebnis des Verfahrens ausgegeben werden. Alternativ ist auch möglich, anhand der Verschiebungsfunktion eine Korrektur der Positionswerte durchzuführen und die Form der abgetasteten Fläche anhand des korrigierten Satzes von Positionsdaten zu ermitteln.

Mit der Verschiebungsfunktion angenäherte Positionswerte können dazu anhand der Verschiebungsfunktion korrigiert werden, so dass sich ein korrigierter Satz von Positionswerten ergibt. Es kann eine Flächenfunktion ermittelt werden, die den korrigierten Satz von Positionswerten annähert. Die Flächenfunktion kann als Repräsentation der Form der abgetasteten Augenstruktur ausgegeben werden. Das Ausgeben kann beispielsweise darin bestehen, dass die Flächenfunktion in mathematischer oder grafischer Darstellung auf einem Display angezeigt wird, dass die Flächenfunktion in einem Speicher abgelegt oder ausgegeben wird, oder dass die Flächenfunktion an ein nachfolgendes Modul zur weiteren Verarbeitung übergeben wird. Diese Aufzählung ist nicht abschließend.

Die Verschiebungsfunktion kann als zeitabhängiges Polynom dargestellt werden. Eine Möglichkeit ist es, den Bewegungsablauf als Überlagerung von translatorischen Bewegungen entlang der drei Achsen eines kartesischen Koordinatensystems zu betrachten und jeder der drei Achsen ein zeitabhängiges Polynom zuzuordnen. Bei kleinen Amplituden können Drehbewegungen als Überlagerungen translatorischer Bewegungen angesehen werden. In einer Weiterbildung der Erfindung können auch Drehbewegungen durch geeignete mathematische Funktionen direkt angenähert werden.

Die Z-Achse des Koordinatensystems kann parallel zu der Richtung des Messstrahls ausgerichtet sein. Der Messstrahl kann parallel zur optischen Achse auf das Auge gerichtet werden. Das zur Z-Achse gehörige Polynom kann von einem höheren Grad sein als das Polynom der X-Achse und/oder das Polynom der Y-Achse. Der Grad des Polynoms der Z-Achse kann beispielsweise zwischen 4 und 8 liegen.

Die Koeffizienten der Verschiebungsfunktion können durch Lösen einer Optimierungsaufgabe gewonnen werden. Kriterium für die Optimierung kann sein, dass die Differenz zwischen der Flächenform, unter Berücksichtigung ihrer aus der Verschiebungsfunktion abgeleiteten zeitlichen Position, und den mit der Messung ermittelten Positionswerten möglichst gering ist. Dieses Kriterium wird vorzugsweise auf alle Positionswerte gleichzeitig angewendet. Das Optimierungsproblem kann beispielsweise nach der Methode der kleinsten Quadrate gelöst werden.

In einer Ausführungsform wird der Satz von Positionswerten in eine Mehrzahl von zeitlich aufeinanderfolgenden Chunks unterteilt. Der Bewegungsablauf während der Zeitdauer eines Chunks kann durch eine Abschnittsfunktion beschrieben werden, wobei die Abschnittsfunktion in der gleichen Weise ermittelt werden kann, wie es oben für die Verschiebungsfunktion beschrieben ist. In einer Ausführungsform wird für jeden Chunk eine Abschnittsfunktion ermittelt. Die Verschiebungsfunktion kann sich durch Aneinanderreihung der Abschnittsfunktionen ergeben. Die Verschiebungsfunktion kann eine stückweise polynomische Funktion (Spline) sein.

Für das Zusammensetzen der Verschiebungsfunktion aus Abschnittsfunktionen kann eine Regularisierung vorgegeben werden, um sicherzustellen, dass die einfachste oder plausibelste Form gefunden wird, mit der die Form der Augenstruktur und die zeitabhängige Translation beschrieben werden können. Die Regularisierung kann einen oder mehrere der nachfolgenden Aspekte umfassen. Die Position von einer Abschnittsfunktion, beispielsweise von der zeitlich ersten Abschnittsfunktion, kann innerhalb des Raums fixiert werden, um zu verhindern, dass der gesamte Satz von Positionswerten innerhalb des Raums verschoben werden kann, ohne dass sich der Näherungsfehler ändert. Die Verschiebungsfunktion kann so regularisert werden, dass sich über alle Abschnittsfunktionen hinweg eine mittlere Verschiebung von null ergibt. Die Verschiebungsfunktion kann so regularisiert sein, dass der Übergang zwischen zwei zeitlich benachbarten Abschnittsfunktionen stetig ist. Die Verschiebungsfunktion kann so regularisiert sein, dass sich in der ersten Ableitung der Verschiebungsfunktion ein stetiger Übergang zwischen zwei zeitlich benachbarten Abschnittsfunktionen ergibt.

Die Flächenform, mit der die Positionswerte angenähert werden, kann ebenfalls durch eine mathematische Funktion dargestellt werden. Ein Beispiel für eine Augenstruktur, auf die sich die Positionswerte beziehen können, ist die Oberfläche der Cornea. Eine mögliche Anwendung des erfindungsgemäßen Verfahrens ist es, dass die Flächenform einer Topographie der Cornea entspricht. Die Cornea, wie auch andere Strukturen des Auges, bei denen das Verfahren angewendet werden kann, haben eine kreisrunde Form. Als mathematische Funktion zur Darstellung der Augenstruktur können deswegen Zernike-Polynome verwendet werden, bei denen es sich um innerhalb des Einheitskreises definierte Polynome handelt. Durch Skalierung kann die mit den Zernike-Polynomen abgedeckte Fläche an die Größe der untersuchten Augenstruktur angepasst werden.

Für die Durchführung der Bewegungskorrektur ist es nicht erforderlich, die Flächenform in der höchstmöglichen mathematischen Genauigkeit darzustellen. Ausreichen kann es, die Flächenform durch Zernike-Polynome niederer Ordnung abzubilden, beispielsweise bis zur vierten Ordnung, bis zur sechsten Ordnung oder bis zur achten Ordnung.

Zur Ermittlung der Flächenform kann ein Optimierungsproblem formuliert werden, bei dem die Flächenform und die Verschiebungsfunktion gemeinsam variiert werden. Wird die Flächenform durch Zernike-Polynome beschrieben, so können die Koeffizienten der Zernike-Polynome auf diese Weise ermittelt werden. Es kann einen einheitlichen Optimierungsvorgang geben, innerhalb dessen sowohl die Koeffizienten der Polynome der Verschiebungsfunktion als auch die Koeffizienten der Zernike-Polynome ermittelt werden. Es wird also mit einem Optimierungsschritt eine Annäherung sowohl im Hinblick auf die Flächenform als auch im Hinblick auf den Bewegungsablauf während des Messzeitraums durchgeführt.

Der Messstrahl, mit dem das Auge gescannt wird, kann ein OCT-Messstrahl sein. Bei OCT-Messungen wird kurzkohärentes OCT-Licht in einen Objektstrahlengang und einen Referenzstrahlengang aufgespalten. Der Objektstrahlengang wird als Messstrahl auf das Objekt geleitet. Von dem Objekt zurückgestreute Anteile des OCT-Lichts werden mit dem Licht des Referenzstrahlengangs zur Interferenz gebracht. Aus dem Interferenzmuster kann auf Streuzentren in dem Objekt geschlossen werden. Die OCT-Lichtquelle kann eine Swept-Source-Lichtquelle sein.

Mit dem Messstrahl kann eine Mehrzahl von A-Scans gewonnen werden. Als A-Scan wird eine Messung bezeichnet, die sich in Richtung des Messstrahls in die Tiefe des Objekts erstreckt. Aus einem solchen A-Scan kann eine Information über die axiale Position einer gesuchten Augenstruktur abgeleitet werden. Ist beispielsweise die gesuchte Augenstruktur die Oberfläche der Cornea, so kann diese Information aus dem A-Scan gewonnen werden. Eine solche aus einem A-Scan abgeleitete Information über die axiale Position einer Augenstruktur ergibt zusammen mit der Position des A-Scans einen Positionswert der Augenstruktur. Durch eine Mehrzahl von A-Scans kann der erfindungsgemäße Satz von Positionswerten der Augenstruktur gewonnen werden. Der Satz von Positionswerte bildet eine Punktwolke, bei der jeder Punkt auf einer Oberfläche der Augenstruktur liegt.

Vor dem Ermitteln der Verschiebungsfunktion und gegebenenfalls der mathematischen Funktion der Flächenform können unplausible Messwerte aus dem Satz von Positionswerten gestrichen werden. Bei der Messung am Auge ist es beispielsweise möglich, dass der Patient während der Messung blinzelt und der Messstrahl deswegen auf das Augenlid auftritt, statt auf die Oberfläche der Cornea. Die Chunks können so definiert sein, dass sie sich über eine bestimmte Zeit erstrecken, so dass im Ausgangspunkt in allen Chunks die gleiche Anzahl von Positionswerte enthalten ist. Nach dem Streichen von unplausiblen Messwerten kann die Zahl der Positionswerte sich zwischen den Chunks unterscheiden. Auch leere Chunks, in denen keine Positionswerte enthalten sind, können auf diese Weise entstehen. Leere Chunks werden beim Zusammensetzen der Verschiebungsfunktion aus Abschnittsfunktionen berücksichtigt, indem zwei Chunks, zwischen denen ein leerer Chunk angeordnet ist, nicht als benachbarte Chunks gelten.

Das Abtastmuster, mit dem der Messstrahl das Auge abtastet, kann so gewählt sein, dass in jedem Chunk Messdaten von der gesamten untersuchten Augenstruktur enthalten sind. Mit anderen Worten kann für jeden der Chunks gelten, dass innerhalb der mit dem Messstrahl überstrichenen Fläche jeder Kreis, der eine Fläche von mehr als 10 %, vorzugsweise von mehr als 5 %, weiter vorzugsweise von mehr als 2 % innerhalb der überstrichenen Fläche abdeckt, wenigstens einen Messwert enthält. Dies gilt für den Zustand, bevor unplausible Messwerte gestrichen wurden.

Das Scannen des Auges mit dem Messstrahl kann so durchgeführt werden, dass der Messstrahl in seitlicher Richtung abgelenkt wird, so dass er eine Kurve auf der Oberfläche des Auges beschreibt. Entlang der Kurve kann die gewünschte Sequenz von A-Scans aufgenommen werden.

Die Kurve kann so gestaltet sein, dass abrupte Richtungsänderungen, die eine hohe Belastung für die Scaneinrichtung darstellen, vermieden werden. Die Kurve kann beispielsweise die folgende Form haben.

$$P_{\mathrm{SP}}(t) = \begin{pmatrix} x_{\mathrm{SP}}(t) \\ y_{\mathrm{SP}}(t) \end{pmatrix} = R_{\mathrm{SP}} \sin(a\,\omega\,t) \begin{pmatrix} \sin(b\,\omega\,t) \\ \cos(b\,\omega\,t) \end{pmatrix}$$

Dabei ist $R_{\mathrm{SP}}$ der Radius der abgetasteten Fläche und $\omega = 2\pi/T$ die aus der Periodendauer T abgeleitete Basisfrequenz der Abtastung. Als Periode T wird der Zeitraum bezeichnet, innerhalb dessen das Abtastmuster einmal durchlaufen wird. Durch die Skalare a und b wird das Verhältnis zwischen den Frequenzen definiert. Der Radius $R_{\mathrm{SP}}$ der abgetasteten Fläche kann beispielsweise zwischen 2 mm und 8 mm, vorzugsweise zwischen 3 mm und 6 mm liegen. Der Skalar b kann um einen Faktor zwischen 1,2 und 4, vorzugsweise zwischen 1,5 und 3 größer sein als der Skalar a. Der Skalar a kann beispielsweise zwischen 10 und 20, der Skalar b zwischen 20 und 40 liegen. Die Periodendauer T, innerhalb derer das Abtastmuster einmal durchlaufen wird, kann beispielsweise zwischen 0,2 Sekunden und 1 Sekunde liegen. Wird die Kurve mit konstanter Geschwindigkeit $\omega$ durchlaufen, so sind die aufgenommenen Positionswerte in bestimmten Bereichen der abgetasteten Struktur dichter und in anderen Bereichen weniger dicht verteilt. In einer Ausführungsform der Erfindung wird die Geschwindigkeit $\omega$ während des Durchlaufens variiert, um eine bessere Gleichverteilung der Positionswerte auf der abgetasteten Struktur zu erreichen.

Beim Scannen des Auges mit dem Messstrahl kann die Kurve mehrfach durchlaufen werden, beispielsweise zwischen zweimal und zehnmal. In einer Ausführungsform wird die Kurve entlang dem identischen Weg mehrfach durchlaufen, wie es beispielsweise möglich ist, wenn der Anfangspunkt und der Endpunkt der Kurve identisch sind. Umfasst sind auch Ausführungsformen, in denen die Kurven dem Muster nach identisch sind, aber relativ zueinander verschoben oder rotiert sind.

Von Vorteil ist es, wenn die Kurve, die der Messstrahl auf der abgetasteten Augenstruktur beschreibt, eine größere Anzahl von Kreuzungspunkten hat. Die Anzahl der Kreuzungspunkte kann beispielsweise größer sein als 50, vorzugsweise größer sein als 100, weiter vorzugsweise größer sein als 400. Dies gilt für ein einmaliges Durchlaufen der Kurve. Bei mehrmaligem Durchlaufen der Kurve kann sich in die Anzahl der Kreuzungspunkt der entsprechend erhöhen. Solche Kreuzungspunkte können beim Ermitteln der Verschiebungsfunktion hilfreich sein. In diesem Zusammenhang ist es weiter von Vorteil, wenn die Positionswerte so aufgenommen werden, dass sie die Kreuzungspunkte möglichst gut treffen. Bei einem Durchlauf der Kurve können beispielsweise zwischen 2000 und 50.000 A-Scans, vorzugsweise zwischen 10.000 und 20.000 A-Scans aufgenommen werden. Ein Chunk kann sich beispielsweise über 1000 bis 25.000 A-Scans, vorzugsweise 5000 bis 10.000 A-Scans erstrecken. Der Satz von Positionswerte kann beispielsweise in 4 bis 20, vorzugsweise 5 bis 10 Chunks unterteilt werden.

Der Ablauf des erfindungsgemäßen Verfahrens wird nachfolgend anhand eines Beispiels geschildert, bei dem die Verschiebungsfunktion und die Flächenform gleichzeitig angenähert werden und bei dem die Verschiebungsfunktion als stückweises Polynom aus einer Mehrzahl von Abschnittsfunktionen zusammengesetzt ist. Die nachfolgend genannten Schritte können im Rahmen der Erfindung einzeln oder in Kombination angewendet werden können. Als Eingangsgröße für das Optimierungsverfahren kann der mit dem Messstrahl ermittelte Satz von Positionswerten der Augenstruktur verwendet werden, wobei die nicht plausiblen Messwerte vorzugsweise zuvor ausgeschlossen wurden.

Der Satz von Positionswerten liegt vor in Form von Koordinaten $P_n = (x_n, y_n, z_n)^T$ und Zeitstempeln $t_n$ mit $n = 0, 1, ..., N^{t-1}$, wobei $N_t$ die Gesamtzahl der Punkte bezeichnet. Der Satz von Positionswerten wird aufgespalten in eine Mehrzahl von Chunks, wobei die Chunks sich beispielsweise über einen Zeitraum zwischen 0,05 s und 0,2 s erstrecken können. Die mit den einzelnen Chunks abgedeckte Zeitspanne kann identisch sein. Die in den Chunks enthaltene Anzahl von Positionswerten kann nach dem Streichen nicht-plausibler Messwerte voneinander abweichen. Chunks, die gänzlich frei von plausiblen Positionswerten sind, können komplett gestrichen und bei dem weiteren Optimierungsverfahren nur noch insoweit berücksichtigt werden, dass zwei Chunks zwischen denen einer leerer Chunk gestrichen wurde, nicht als benachbarte Chunks gelten. Es ergibt sich eine Anzahl von Z* gültiger Chunks.

Die stückweise polynomische Verschiebung der Chunks kann definiert sein durch folgende Funktion f(t):

$$f(t) = \begin{pmatrix} f_x(t) \\ f_y(t) \\ f_z(t) \end{pmatrix} = \sum_{i=0}^{Z^*-1} \mathrm{boxchar}_i(t) \begin{pmatrix} \alpha^x_{i\,0}\,1 + \alpha^x_{i\,1}\,(t - t^m_i)^1 + \ldots + \alpha^x_{i\,O_x}\,(t - t^m_i)^{O_x} \\ \alpha^y_{i\,0}\,1 + \alpha^y_{i\,1}\,(t - t^m_i)^1 + \ldots + \alpha^y_{i\,O_y}\,(t - t^m_i)^{O_y} \\ \alpha^z_{i\,0}\,1 + \alpha^z_{i\,1}\,(t - t^m_i)^1 + \ldots + \alpha^z_{i\,O_z}\,(t - t^m_i)^{O_z} \end{pmatrix}$$

Dabei bezeichnet $t_i^m$ die Durchschnittszeit der Positionswerte im Chunk i, und $\alpha$ sind die Koeffizienten der einzelnen Polynome. Die boxchar-Funktion $\mathrm{boxchar}_i(t)$ ist gleich 1, wenn t innerhalb des Chunk-Intervalls i liegt, und ist gleich 0, wenn t außerhalb dieses Intervalls liegt. $O_x = 6$, $O_y = 6$ und $O_z = 8$ bezeichnet den Grad der Verschiebungspolynome in der betreffenden Achse. Als Z-Achse wird die zu dem Messstrahl parallele Achse bezeichnet. Die X-Achse und Y-Achse sind orthogonal dazu.

Die Koeffizienten der Verschiebungspolynome werden bestimmt durch Lösen des folgenden Näherungsproblems der kleinsten mittleren Quadrate:

$$\underset{\beta,\, \mathbf{p}}{\text{minimize}} \quad \left\| \begin{pmatrix} \mathbf{Z} & \mathbf{M} \\ \mathbf{0} & \mathbf{C} \end{pmatrix} \begin{pmatrix} \boldsymbol{\beta} \\ \boldsymbol{\alpha} \end{pmatrix} - \begin{pmatrix} \mathbf{z} \\ \mathbf{0} \end{pmatrix} \right\|_2^2$$

Dabei ist $\beta$ ein Reihenvektor mit den Zernike-Koeffizienten und $\alpha$ ein Reihenvektor mit den Koeffizienten der Verschiebungspolynome. Die Spalten der Matrix Z enthalten die Zernike-Polynome, die an den x,y-Koordinaten aufgenommen wurden, während z die z-Koordinaten der Positionswerte repräsentiert. Die Matrix M enthält die polynomischen Basisfunktionen für die Verschiebung in jedem Chunk und $\alpha$ die entsprechenden Koeffizienten:

$$\mathbf{M} = \begin{pmatrix} \mathbf{M}_0 & \mathbf{0} & \ddots & \mathbf{0} \\ \mathbf{0} & \mathbf{M}_1 & \ddots & \mathbf{0} \\ \ddots & \ddots & \ddots & \ddots \\ \mathbf{0} & \mathbf{0} & \ddots & \mathbf{M}_{Z^*-1} \end{pmatrix}$$

$$\alpha = \begin{pmatrix} \alpha_0 & \alpha_1 & \ldots & \alpha_{Z^*-1} \end{pmatrix}^T$$

$M_i$ enthält die Verschiebungs-Basisfunktion in allen drei Achsen und $\alpha_i$ sind die Koeffizienten der Verschiebungs-Polynome in allen drei Achsen für den spezifischen Chunk$_i$.

$$\mathbf{M}_i = \begin{pmatrix} \mathbf{M}_i^x & \mathbf{M}_i^y & \mathbf{M}_i^z \end{pmatrix}$$

$$\alpha_i = \begin{pmatrix} \alpha_i^x & \alpha_i^y & \alpha_i^z \end{pmatrix}^T = \begin{pmatrix} \alpha_{i\,0}^x & \cdots & \alpha_{i\,O_x}^x & \alpha_{i\,0}^y & \cdots & \alpha_{i\,O_y}^y & \alpha_{i\,0}^z & \cdots & \alpha_{i\,O_z}^z \end{pmatrix}^T$$

Die Spalten von $M_i^x$, $M_i^y$, $M_i^z$ repräsentieren die Verschiebungs-Basisfunktionen in den betreffenden Richtungen. Die Verschiebungs-Basisfunktionen für die x- und y-Richtung werden geschätzt auf Basis eines anfänglichen Zernike-Fits auf die Wolke von Positionswerte. Die Ordnung des Zernike-Fits kann beispielsweise zwischen 4 und 8, vorzugsweise bei 6 liegen. Jeder Eintrag wird ermittelt durch partielle Ableitung der Zernike-Oberfläche bei den x-,y-Koordinaten der betreffenden Punkte n und Ordnung m.

$$\mathbf{M}_{i\,n,m}^x = \frac{d}{dx} Z(x_n, y_n) \times (t_n - T_i)^m$$

$$\mathbf{M}_{i\,n,m}^y = \frac{d}{dy} Z(x_n, y_n) \times (t_n - T_i)^m$$

Für die Z-Richtung vereinfacht sich dies zu:

$$\mathbf{M}^z_{i\,n,m} = (t_n - T_i)^m$$

Eine Regularisierungs-Matrix C wird eingeführt, um Diskontinuitäten in der Verschiebung und ihrer ersten Ableitung zu bestrafen und um den Offset der Regularisierung zu bestrafen. Das Bestrafen von Diskontinuitäten ist auf benachbarte Chunks beschränkt. Aufeinander folgende Chunks werden als benachbart betrachtet, wenn kein leerer Chunk zwischen ihnen entfernt wurde.

$$C = \begin{pmatrix} \mathbf{R}(t_0^l - t_0^m) & -\mathbf{R}(t_0^l - t_1^m) & 0 & \ddots & 0 & 0 \\ 0 & \mathbf{R}(t_1^c - t_1^m) & -\mathbf{R}(t_1^c - t_2^m) & \ddots & 0 & 0 \\ \ddots & \ddots & \ddots & \ddots & \ddots & \ddots \\ 0 & 0 & 0 & \ddots & -\mathbf{R}(t_{Z^*-3}^c - t_{Z^*-2}^m) & 0 \\ 0 & 0 & 0 & \ddots & \mathbf{R}(t_{Z^*-2}^c - t_{Z^*-2}^m) & -\mathbf{R}(t_{Z^*-2}^c - t_{Z^*-1}^m) \\ \mathbf{S} & 0 & 0 & \ldots & 0 & 0 \end{pmatrix}$$

Die R-Matrizen enthalten die Verschiebungs-Basisfunktionen und ihre ersten Ableitungen an den Schnittpunkten benachbarter Chunks für jede Richtung.

$$\mathbf{R}(t) = \begin{pmatrix} \mathbf{R}^x(t) & 0 & 0 \\ 0 & \mathbf{R}^y(t) & 0 \\ 0 & 0 & \mathbf{R}^z(t) \end{pmatrix}$$

$$\mathbf{R}^x(t) = \begin{pmatrix} 1 & t & t^2 & \ldots & t^{O}_x \\ 0 & 1 & 2t & \ldots & O_x t^{O_x-1} \end{pmatrix}$$

$$\mathbf{R}^y(t) = \begin{pmatrix} 1 & t & t^2 & \ldots & t^{O}_y \\ 0 & 1 & 2t & \ldots & O_y t^{O_y-1} \end{pmatrix}$$

$$\mathbf{R}^z(t) = \begin{pmatrix} 1 & t & t^2 & \ldots & t^{O}_z \\ 0 & 1 & 2t & \ldots & O_z t^{O_z-1} \end{pmatrix}$$

Ohne die Regularisierung wäre das Problem unterbestimmt und die gesamte Wolke von Positionswerte könnte ohne Einfluss auf den Näherungsfehler verschoben werden. Mit der Matrix S wird der Offset des ersten Chunks $\alpha_0 x_0$ $\alpha_0 y_0$ $\alpha_0 z_0$ auf null gesetzt, womit eine Verschiebung der kompletten Oberfläche verhindert wird.

$$\mathbf{S} = s_{\text{reg}} \begin{pmatrix} 1 & 0 & 0 & 0 & \ldots & 0 \\ 0 & 1 & 0 & 0 & \ldots & 0 \\ 0 & 0 & 1 & 0 & \ldots & 0 \end{pmatrix}$$

Der Skalar $s_{\text{reg}}$ definiert die Stärke der Regularisierung.
Nach dem Lösen des Kleinste-Quadrate-Problems, werden die Positionswerte durch Subtraktion der festgestellten Verschiebung von den Punktkoordinaten korrigiert.

$$P_n^* = P_n - f(t_n) \quad \text{for } n = 0, 1, \ldots N^T - 1$$

Nachdem die Verschiebungsfunktion ermittelt wurde, kann eine Qualitätsüberprüfung durchgeführt werden. Wurde das Optimierungsverfahren auf Basis von N Chunks durchgeführt, so können durch Ausschließen jeweils einzelner Chunks N verschiedene Subsets gebildet werden, die jeweils N-1 Chunks umfassen.

Für jedes der Subsets kann die Koeffizienten-Standardabweichung einer Zernike-Rekonstruktion bestimmt werden. Der Grad der Zernike-Rekonstruktion kann höher sein als der Grad der Zernike-Rekonstruktion beim Ermitteln der Verschiebungsfunktion, insbesondere kann der Grad um 1 erhöht sein. Beispielsweise kann eine Zernike-Rekonstruktion siebten Grades durchgeführt werden. Ist die kleinste Koeffizienten-Standardabweichung für eines der Subsets kleiner als die Koeffizienten-Standardabweichung über alle Chunks, so kann das betreffende Subset an die Stelle der vorherigen Gesamtheit der Daten treten. In Folge der Qualitätsüberprüfung kann also mit einem reduzierten Satz von Positionswerte fortgefahren werden, in dem die Positionswerte eines Chunks gestrichen sind. Die Qualitätsüberprüfung kann iterativ fortgesetzt, bis die Koeffizienten-Standardabweichung der (verbleibenden) Gesamtdaten kleiner ist als die Koeffizienten-Standardabweichung von jedem der Subsets.

Auf Basis der Positionswerte, die anhand der Verschiebungsfunktion korrigiert wurden, kann die Form der Augenstruktur (beispielsweise die Topographie der Cornea-Oberfläche) rekonstruiert werden. Dafür kann eine Zernike-Rekonstruktion auf die korrigierten Positionswerte angewendet werden. Der Grad der Zernike-Rekonstruktion kann höher sein als der Grad der Zernike-Polynome, mit dem die Flächenform beim Ermitteln der Verschiebungsfunktion dargestellt wird. Beispielsweise kann eine Zernike-Rekonstruktion mit Zernike-Polynome bis zum 10. Grad durchgeführt werden.

Daraus können dann weitere für den Nutzer wertvolle Informationen abgeleitet werden, wie beispielsweise Krümmungs-Karten der Cornea-Vorderseite und -Rückseite sowie eine Kartendarstellung der Hornhautdicke. Auch bewegungskorrigierte Schnittbilder können berechnet werden, oder andere Augenstrukturen, wie beispielsweise die Augenlinse, können bewegungsfrei vermessen werden.

Die Erfindung betrifft außerdem ein zugehöriges Messsystem. Das Messsystem umfasst einen Messstrahl und eine Scaneinrichtung, um den Messstrahl in seitlicher Richtung abzulenken. Mit einem Detektor wird aus einem von dem Messstrahl erzeugten Signal ein Satz von Positionswerten einer Augenstruktur bereitgestellt, wobei die Positionswerte jeweils mit einem Zeitstempel innerhalb eines Messzeitraums versehen sind. Eine Auswerteeinheit ist dazu ausgelegt, eine Flächenform zu ermitteln und eine Verschiebungsfunktion zu ermitteln, wobei die Verschiebungsfunktion einen Bewegungsablauf während des Messzeitraums darstellt, und wobei die Flächenform und die Verschiebungsfunktion derart ermittelt werden, dass die Positionswerte durch die Flächenform angenähert werden, wenn die Flächenform gemäß der Verschiebungsfunktion bewegt wird.

Das Messsystem kann mit weiteren Merkmalen fortgebildet werden, die im Zusammenhang des erfindungsgemäßen Verfahrens beschrieben sind. Das Verfahren kann mit weiteren Merkmalen fortgebildet werden, die im Zusammenhang des erfindungsgemäßen Messsystems beschrieben sind.

Die Erfindung wird nachfolgend unter Bezugnahme auf die beigefügten Zeichnungen anhand vorteilhafter Ausführungsformen beispielhaft beschrieben. Es zeigen:

Fig. 1:     eine schematische Darstellung eines erfindungsgemäßen Messsystems;

Fig. 2:     eine erfindungsgemäße Abtastkurve;

Fig. 3:     die zu Fig. 2 gehörige Ablenkung der Scaneinrichtung in X-Richtung und Y-Richtung;

Fig. 4:     die tatsächliche Verschiebung eines Objekts in X-Richtung, Y-Richtung und Z-Richtung;

Fig. 5:     eine zu Fig. 4 gehörige Verschiebungsfunktion, die mit dem erfindungsgemäßen Verfahren ermittelt wurde;

Fig. 6:     eine grafische Darstellung eines Satzes von Positionswerte.

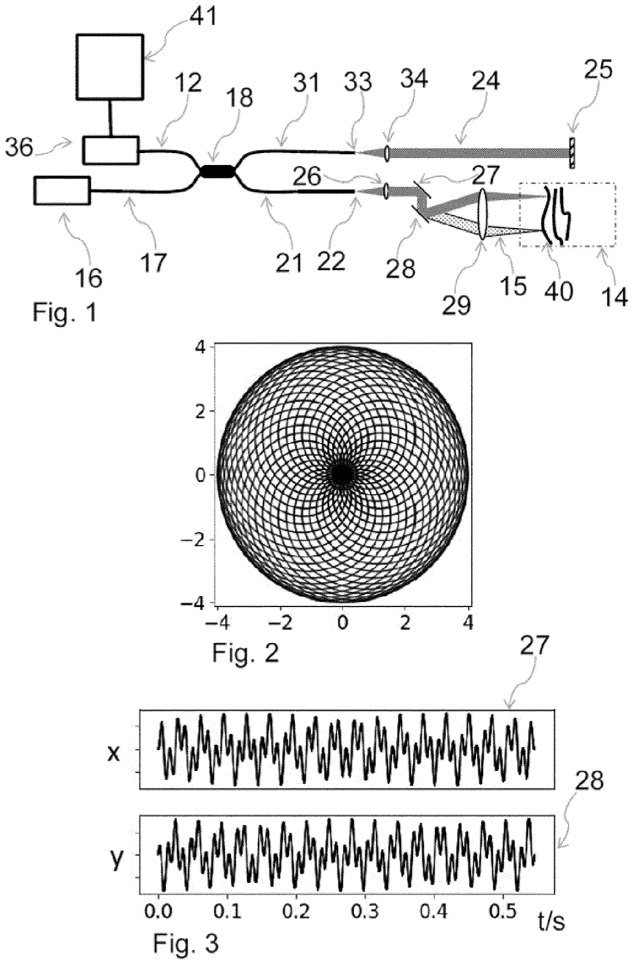

Mit einem in Fig. 1 gezeigten OCT-Messsystem wird ein Messobjekts 14 in Form eines menschlichen Auges abgetastet. Indem OCT-Licht 15 auf das Messobjekt 14 gerichtet wird, wird eine Bildinformation gewonnen, die sich entlang der Achse des OCT-Strahls in die Tiefe des Messobjekts 14 erstreckt (A-Scan). Indem der OCT-Strahl in einer dazu senkrechten Richtung über das Messobjekt 14 gescannt wird, kann aus einer Vielzahl von A-Scans ein dreidimensionales Bild des Messobjekts 14 zusammengesetzt werden. Während der Zeitspanne, die das Scannen des Auges in Anspruch nimmt, kann das Auge sich bewegen, was sich negativ auf die Qualität der Bildinformation auswirken kann.

Das OCT-System umfasst eine OCT-Lichtquelle 16, die als Swept-Source-Lichtquelle ausgebildet ist. Die Swept-Source-Lichtquelle 16 erzeugt schmalbandiges Licht, das spektral durchstimmbar ist. Zu jedem Moment wird also schmalbandiges Licht ausgesendet, dessen Frequenz sich mit der Zeit ändert, so dass die Swept-Source-Lichtquelle während einer Durchstimmzeit über einen Frequenzbereich durchgestimmt wird.

Das von der OCT-Lichtquelle 16 abgegebene OCT-Licht 15 wird in einen ersten Lichtleiter 17 eingespeist, der sich zu einem Faserkoppler 18 erstreckt. In dem Faserkoppler 18 wird das OCT-Licht 15 aus dem ersten Lichtleiter 17 in einen Objektstrahlengang 23 und einen Referenz-Strahlengang 24 aufgespalten. Der Objektstrahlengang 23 erstreckt sich entlang einem Objektarm bis zu dem Messobjekt 14. Der Referenzstrahlengang 24 erstreckt sich entlang einem Referenzarm bis zu einem Referenzspiegel 25.

Der Objektarm umfasst einen zweiten Lichtleiter 21, der sich von dem Faserkoppler 18 bis zu einem Austrittsende 22 erstreckt. An dem Austrittsende 22 tritt der Objektstrahlengang 23 in divergentem Zustand aus dem zweiten Lichtleiter 21 aus und wird mit einer Kollimationslinse 26 in einen kollimierten Zustand gebracht.

Eine Scaneinrichtung umfasst zwei Scanspiegel 27, 28, die um zwei zueinander orthogonale Achsen schwenkbar sind. Der Objektstrahlengang 23 wird über die Scaneinrichtung 27, 28 zu einem Objektiv 29 geleitet. Der Objektstrahlengang 23 tritt durch das Objektiv 29 hindurch und wird im Bereich des Messobjekts 14 fokussiert.

Durch Schwenken der Scanspiegel 27, 28 ändert sich die Richtung, unter der der Objektstrahlengang 23 auf das Objektiv 29 auftrifft. Da der zweite Scanspiegel 28 im Brennpunkt des Objektivs 29 angeordnet ist, erstreckt der Strahlengang 23 sich zwischen dem Objektiv 29 und dem Messobjekt 14 unabhängig von der Stellung des Scaneinrichtung 27, 28 parallel zur optischen Achse des Objektivs 29.

Von dem Messobjekt 14 zurückgeworfenes OCT-Licht bewegt sich mit entgegengesetzter Ausbreitungsrichtung entlang dem Objektarm 19 zurück bis zu dem Faserkoppler 18.

Der Referenzarm umfasst einen dritten Lichtleiter 31, der sich von dem Faserkoppler 18 über einen zweiten Polarisationssteller 32 bis zu einem Austrittsende 33 erstreckt. Der in divergentem Zustand aus dem Austrittsende 33 austretende Referenzstrahlengang 24 trifft auf eine Kollimationslinse 34. Von der Kollimationslinse 34 breitet der Referenzstrahlengang 24 sich in kollimiertem Zustand bis zu dem Referenzspiegel 25 aus. Das von dem Referenzspiegel 25 reflektierte OCT-Licht läuft mit entgegengesetzter Ausbreitungsrichtung entlang dem Referenzarm 20 zurück zu dem Faserkoppler 18.

Der Referenzspiegel 25 ist so angeordnet, dass der optische Weg zwischen dem Faserkoppler 18 und dem Referenzspiegel 25 im Referenzarm 20 genauso lang ist wie der optische Weg im Objektarm 19 zwischen dem Faserkoppler 18 und einem Referenzpunkt im Messobjekt 14. Da das OCT-Licht entlang dem Objektarm 19 und dem Referenzarm 20 denselben optischen Weg zurückgelegt hat, entsteht ein Interferenzsignal, wenn der Objektstrahlengang 23 und der Referenzstrahlengang 24 in dem Faserkoppler 18 wieder zusammengeführt werden. Das Interferenzsignal ist desto stärker, je mehr OCT-Licht von einer bestimmten Struktur innerhalb des Messobjekts 14 zurückgeworfen wird. Durch Auswerten des Interferenzsignals können Streuzentren innerhalb des Messobjekts 14 identifiziert werden.

Ist ein Streuzentrum gerade an dem Referenzpunkt des Objektstrahlengangs angeordnet, so sind der optische Weg des Objektstrahlengangs 23 und des Referenzstrahlengangs 24 exakt gleich lang, so dass sich ein stehendes Interferenzsignal ergibt. Ist das Streuzentrum von dem Referenzpunkt entfernt, so oszilliert das Interferenzsignal (in spektraler Darstellung), wobei die Frequenz je größer wird, desto größer der Abstand zum Referenzpunkt ist.

Über einen vierten Lichtleiter 12 wird das Interferenzsignal zu einem Detektor 36 geleitet. Mit dem Detektor 36 wird das Interferenzsignal aufgenommen und in eine ortsaufgelöste Bildinformation umgerechnet.

In Fig. 2 ist eine Kurve gezeigt, entlang derer sich der Messstrahl 15 in X-Richtung und in Y-Richtung unter der Kontrolle der Scaneinrichtung 27, 28 relativ zu dem Auge 14 bewegt. Der Radius des in Fig. 2 überstrichenen Kreises ist 4 mm. Mit einem Kreisdurchmesser von 8 mm kann die Cornea eines menschlichen Auges abgedeckt werden.

Die Kurve gemäß Fig. 2 kann mit dem Messstrahl 15 abgefahren werden, indem die Scaneinrichtung 27, 28 gemäß den in Fig. 3 gezeigten Kurven in X-Richtung und in Y-Richtung angesteuert wird. Die Ansteuerung erfolgt gemäß folgender Funktion, deren Parameter oben näher erläutert sind.

$$P_{SP}(t) = \begin{pmatrix} x_{SP}(t) \\ y_{SP}(t) \end{pmatrix} = R_{SP} \sin(a\,\omega\,t) \begin{pmatrix} \sin(b\,\omega\,t) \\ \cos(b\,\omega\,t) \end{pmatrix}$$

Diese Kurve hat den Vorteil, dass sie schnell abgefahren werden kann, da abrupte Richtungswechsel der Scanspiegel 27, 28 vermieden werden. Ein einmaliger Durchlauf der Kurve erstreckt sich über 0,546 Sekunden.

Werden die A-Scans mit einer Frequenz von 30 kHz aufgenommen, so ergeben sich 16.384 A-Scans pro Durchlauf der in Fig. 2 gezeigten Kurve. Im vorliegenden Ausführungsbeispiel wird die Kurve für die Aufnahme eines Satzes von Positionswerten viermal durchlaufen, so dass insgesamt 65.536 A-Scans aufgenommen werden, wobei die Messung sich über eine Dauer von 2,184 Sekunden erstreckt.

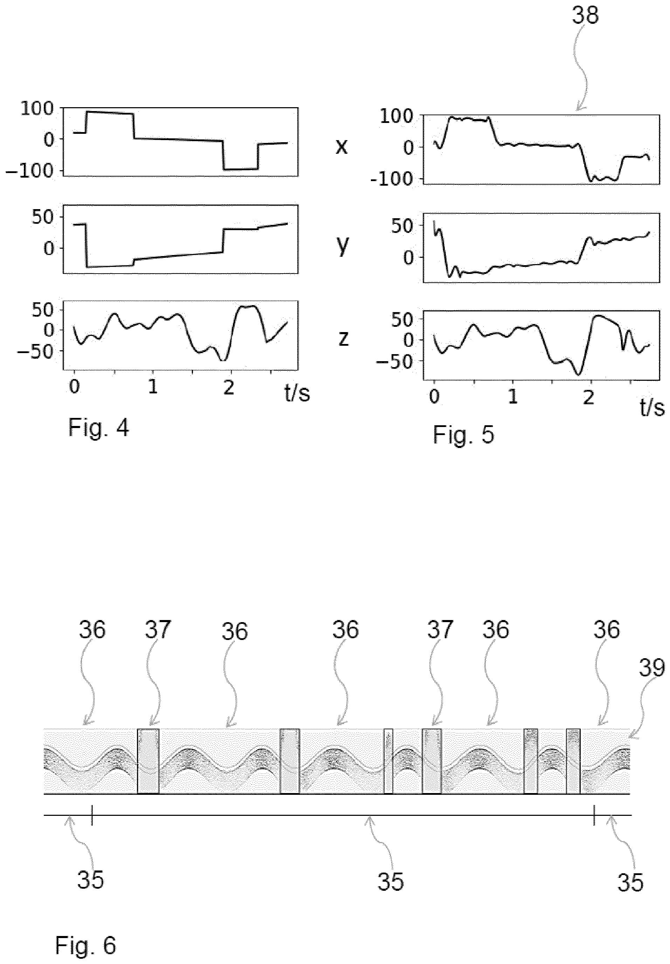

In Fig. 6 ist die während einer Messung mit dem Detektor 36 ermittelte Bildinformation grafisch dargestellt, indem einige der 65.536 A-Scans so nebeneinander angeordnet sind, dass sich ein durchgehendes Schnittbild ergibt. Die Messung wird in 16 zeitlich aufeinanderfolgende Chunks 35 aufgeteilt, von denen einer in Fig. 6 vollständig und zwei benachbarte ausschnittsweise dargestellt sind.

In der Messung gibt es große Abschnitte 36 mit plausiblen Messwerten und kleinere Abschnitte 37 mit nicht plausiblen Messwerten, bei denen das Auge durch das Augenlid bedeckt ist. Die Messwerte der nicht plausiblen Abschnitte 37 werden aus den Chunks 35 gelöscht. Es verbleibt eine reduzierte Anzahl von A-Scans, in denen jeweils eine Ortsinformation über die Position der Cornea-Oberfläche in Z-Richtung entnommen werden kann. Zusammen mit der Position des Messstrahls 15 in X-Richtung und Y-Richtung ergibt sich ein Satz von Positionswerte der Cornea-Oberfläche.

Mit dem erfindungsgemäßen Verfahren werden aus den Positionswerten eine Flächenform und eine Verschiebungsfunktion 38 in Form eines stückweisen Polynoms ermittelt. Die einzelnen Verfahrensschritte zur Ermittlung der Verschiebungsfunktion 38 und der Flächenform sind oben näher erläutert. Mit der Verschiebungsfunktion 38 wird die Beziehung zwischen den Positionswerten der verschiedenen Chunks 35 als translatorische Verschiebung beschrieben.

In Fig. 5 ist ein Beispiel einer solchen Verschiebungsfunktion 38 in X-Richtung, Y-Richtung und in Z-Richtung dargestellt. In den drei übereinander angeordneten Graphen ist jeweils die Translation in Mikrometern über der Zeit in Sekunden aufgetragen. Die Verschiebungskurve 38 stammt von einem Versuch, bei dem die tatsächliche Bewegung des Messobjekts 14 den in Fig. 4 gezeigten Verlauf hatte. Es zeigt sich, dass die Verschiebungen 38 den tatsächlichen Verlauf der Bewegung gut wiedergeben.

Aus der Verschiebungsfunktion 38 kann abgelesen werden, in welcher Weise das Auge während der Aufnahme der Positionswerte translatorisch gegeneinander verschoben wurde. Die Positionswerte können gemäß der Verschiebungsfunktion relativ zueinander verschoben werden, so dass die mit der Verschiebungsfunktion 38 ermittelte Translation ausgeglichen wird. Es ergibt sich ein bewegungskorrigierter Satz von Positionswerte, der die Oberfläche der Cornea beschreibt.

Aus diesem Satz von Positionswerte kann die Topographie der Cornea mit einer Zernike-Rekonstruktion höherer Ordnung (zum Beispiel 10. Ordnung) rekonstruiert werden. Es können bewegungskorrigierte Schnittbilder berechnet werden, oder andere Augenstrukturen, wie beispielsweise die Augenlinse, bewegungsfrei vermessen werden.

**Patentansprüche**

1. Verfahren zum Durchführen einer Bewegungskorrektur bei Messungen an einem menschlichen Auge (14), mit folgenden Schritten:

   a. Scannen des Auges (14) mit einem Messstrahl (15), um einen Satz von Positionswerten (39) der Augenstruktur(40) zu gewinnen, wobei das Scannen sich über einen Messzeitraum erstreckt und wobei die Positionswerte (39) jeweils mit einem Zeitstempel innerhalb des Messzeitraums versehen sind;
   b. Ermitteln einer Flächenform und Ermitteln einer Verschiebungsfunktion (38), wobei die Verschiebungsfunktion (38) einen Bewegungsablauf während des Messzeitraums darstellt, und wobei die Flächenform und die Verschiebungsfunktion (38) derart ermittelt werden, dass die Positionswerte (39) durch die Flächenform angenähert werden, wenn die Flächenform gemäß der Verschiebungsfunktion (38) bewegt wird.

2. Verfahren nach Anspruch 1, **dadurch gekennzeichnet, dass** die Positionswerte (39) anhand der Verschiebungsfunktion (38) korrigiert werden.

3. Verfahren nach Anspruch 1 oder 2, **dadurch gekennzeichnet, dass** eine bewegungskorrigierte Topographie der Cornea ermittelt wird.

4. Verfahren nach einem der Ansprüche 1 bis 3, **dadurch gekennzeichnet, dass** die Form der Augenstruktur (40) durch eine Summe von Zernike-Polynomen dargestellt wird.

5. Verfahren nach Anspruch 4, **dadurch gekennzeichnet, dass** die Koeffizienten der Verschiebungsfunktion (39) und die Koeffizienten der Zernike-Polynome durch Lösen einer einheitlichen Optimierungsaufgabe ermittelt werden.

6. Verfahren nach einem der Ansprüche 1 bis 5, **dadurch gekennzeichnet, dass** die Verschiebungsfunktion (39) eine Aneinanderreihung von Abschnittsfunktionen, wobei jede Abschnittsfunktion ein zeitabhängiges Polynom ist.

7. Verfahren nach Anspruch 6, **dadurch gekennzeichnet, dass** in jedem Chunk (35) Positionswerte (39) von der gesamten untersuchten Augenstruktur (40) enthalten sind.

8. Verfahren nach Anspruch 6 oder 7, **dadurch gekennzeichnet, dass** die Optimierungsaufgabe regularisiert wird, indem die Verschiebungsfunktion (39) zwischen benachbarten Abschnittsfunktionen als stetig und/oder als in der ersten Ableitung stetig angenommen wird.

9. Verfahren nach einem der Ansprüche 1 bis 8, **dadurch gekennzeichnet, dass** der Messstrahl (15) ein OCT-Messstrahl ist.

10. Verfahren nach einem der Ansprüche 1 bis 9, **dadurch gekennzeichnet, dass** der Messstrahl entlang einer Kurve über die Augenstruktur gescannt wird, wobei die Kurve wenigstens 50, vorzugsweise wenigstens 100, weiter vorzugsweise wenigstens 400 Kreuzungspunkte hat.

11. Verfahren nach Anspruch 10, **dadurch gekennzeichnet, dass** die Kurve mehrfach durchlaufen wird.

12. Messsystem mit einem Messstrahl (15) und einer Scaneinrichtung (27, 28), um den Messstrahl (15) in seitlicher Richtung abzulenken, mit einem Detektor (36), der aus einem von dem Messstrahl (15) erzeugten Signal einen Satz von Positionswerten (39) einer Augenstruktur (14) bereitstellt, wobei die Positionswerte (39) jeweils mit einem Zeitstempel innerhalb eines Messzeitraums versehen sind, und mit einer Auswerteeinheit (41), die dazu ausgelegt ist, eine Flächenform zu ermitteln und eine Verschiebungsfunktion (38) zu ermitteln, wobei die Verschiebungsfunktion (38) einen Bewegungsablauf während des Messzeitraums darstellt, und wobei die Flächenform und die Verschiebungsfunktion (38) derart ermittelt werden, dass die Positionswerte (39) durch die Flächenform angenähert werden, wenn die Flächenform gemäß der Verschiebungsfunktion (38) bewegt wird.

Fig. 1

Fig. 2

Fig. 3

38

x

y

z

Fig. 4

Fig. 5

36    37    36       36    37    36       36

39

35                    35              35

Fig. 6

**Europäisches Patentamt**
**European Patent Office**
**Office européen des brevets**

# EUROPÄISCHER RECHERCHENBERICHT

Nummer der Anmeldung

EP 18 21 0058

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | HANSFORD C. HENDARGO ET AL: "Automated non-rigid registration and mosaicing for robust imaging of distinct retinal capillary beds using speckle variance optical coherence tomography", BIOMEDICAL OPTICS EXPRESS, Bd. 4, Nr. 6, 7. Mai 2013 (2013-05-07), Seite 803, XP055295793, United States ISSN: 2156-7085, DOI: 10.1364/BOE.4.000803 * Zusammenfassung; Abbildungen 1,2,7 * * Abschnitt 3.5; Seite 812 - Seite 813 * * Abschnitt 2; Seite 807 - Seite 808 * ----- | 1-12 | INV. A61B3/102 G06T3/00 |
| A | US 2018/192866 A1 (ABOU SHOUSHA MOHAMED [US] ET AL) 12. Juli 2018 (2018-07-12) * Zusammenfassung * * Absätze [0085] - [0088] * ----- | 1-12 | |
| A | WO 2013/107649 A1 (ZEISS CARL MEDITEC AG [DE]) 25. Juli 2013 (2013-07-25) * das ganze Dokument * ----- | 1-12 | RECHERCHIERTE SACHGEBIETE (IPC) A61B G06T |
| A,D | US 7 365 856 B2 (ZEISS CARL MEDITEC INC [US]) 29. April 2008 (2008-04-29) * das ganze Dokument * ----- | 1-12 | |

Der vorliegende Recherchenbericht wurde für alle Patentansprüche erstellt

| Recherchenort | Abschlußdatum der Recherche | Prüfer |
|---|---|---|
| München | 5. Juni 2019 | Daniel, Christian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 18 21 0058

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-06-2019

| Im Recherchenbericht angeführtes Patentdokument | Datum der Veröffentlichung | Mitglied(er) der Patentfamilie | Datum der Veröffentlichung |
|---|---|---|---|
| US 2018192866 A1 | 12-07-2018 | US 2018192866 A1 | 12-07-2018 |
| | | WO 2018132621 A1 | 19-07-2018 |
| WO 2013107649 A1 | 25-07-2013 | EP 2804519 A1 | 26-11-2014 |
| | | JP 6227560 B2 | 08-11-2017 |
| | | JP 2015504740 A | 16-02-2015 |
| | | US 2013188140 A1 | 25-07-2013 |
| | | US 2016038021 A1 | 11-02-2016 |
| | | WO 2013107649 A1 | 25-07-2013 |
| US 7365856 B2 | 29-04-2008 | EP 1838213 A1 | 03-10-2007 |
| | | JP 4777362 B2 | 21-09-2011 |
| | | JP 2008528954 A | 31-07-2008 |
| | | US 2006164653 A1 | 27-07-2006 |
| | | US 2008221819 A1 | 11-09-2008 |
| | | US 2010245838 A1 | 30-09-2010 |
| | | US 2012140175 A1 | 07-06-2012 |
| | | US 2014240670 A1 | 28-08-2014 |
| | | US 2016106314 A1 | 21-04-2016 |
| | | WO 2006077107 A1 | 27-07-2006 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- US 7365856 B2

- EP 2797493 A1